# EUROPEAN PATENT SPECIFICATION

(11) **EP 1 864 128 B1**
(45) Date of publication and mention of the grant of the patent: **02.12.2009**
(21) Application number: 06719201.3
(22) Date of filing: 19.01.2006
(51) Int. Cl.: G01N 33/52, C12Q 1/04

(54) **TECHNIQUE FOR DETECTING MICROORGANISMS**
TECHNIK ZUM NACHWEIS VON MIKROORGANISMEN
TECHNIQUE POUR DÉTECTER DES MICRO-ORGANISMES

(30) Priority: 30.03.2005 US 94807
(43) Date of publication of application: 12.12.2007
(73) Proprietor: KIMBERLY-CLARK WORLDWIDE, INC., Neenah, WI 54956 (US)
(72) Inventor: MAC DONALD, John, Gavin, Decatur, GA 30033 (US); BORDERS, Richard, A., Marietta, GA 30062 (US)
(74) Representative: Leidescher, Thomas
(86) International application number: PCT/US2006/002250
(87) International publication number: WO 2006/107370

(56) References cited:
- EP-A- 0 446 972
- EP-A- 1 327 874
- WO-A-01/71318
- WO-A-97/08337
- WO-A-2004/057331
- US-A- 4 073 691
- US-A- 5 753 285
- US-A1- 2003 003 593
- US-A1- 2004 161 472
- PONDER JENNIFER B ET AL: "Detection and identification of pathogenic bacteria via colorimetric sensing array." ABSTRACTS OF PAPERS AMERICAN CHEMICAL SOCIETY, vol. 227, no. Part 1, March 2004 (2004-03), page U88, XP008063520 & 227TH NATIONAL MEETING OF THE AMERICAN-CHEMICAL SOCIETY; ANAHEIM, CA, USA; MARCH 28 -APRIL 01, 2004 ISSN: 0065-7727
- PONDER JENNIFER B ET AL: "Rapid evaluation of pathogenic bacteria cultures via colorimetric sensing array." ABSTRACTS OF PAPERS AMERICAN CHEMICAL SOCIETY, vol. 229, no. Part 1, 13 March 2005 (2005-03-13), pages U102-U103, XP008063519 & 229TH NATIONAL MEETING OF THE AMERICAN-CHEMICAL-SOCIETY; SAN DIEGO, CA, USA; MARCH 13 -17, 2005 ISSN: 0065-7727

## Description

### Background of the Invention

The ability to rapidly detect microorganisms is becoming an increasing problem in a wide variety of industries. For instance, food products (e.g., meat) are normally analyzed before, during, or after entry into an establishment. However, no further testing generally occurs before the food product is consumed, leaving the possibility that undetected food-borne pathogens, such as *Salmonella* and *Listeria*, will multiply to an undesirable level during the packaging, transportation, and display of the product. For instance, a temperature increase of less than 3°C may shorten food shelf life by 50% and cause a significant increase in bacterial growth over time. Indeed, spoilage of food may occur in as little as several hours at 37°C based on a total pathogenic and non-pathogenic bacterial load of 10³ colony forming units ("cfu") per gram on food products. Food safety leaders have identified this level as the maximum acceptable threshold for meat products.

A number of devices are known that provide a diagnostic test reflecting either bacterial load or food freshness, including time-temperature indicator devices. To date, none of these devices have been widely accepted due to the technology applied. For instance, wrapping film devices typically require actual contact with the bacteria. If the bacteria are internal to the exterior food surface, however, then an internally high bacterial load on the food does not activate the sensor. Ammonia sensors have also been developed, but are only able to detect bacteria that break down proteins. Because bacteria initially utilize carbohydrates, these sensors have a low sensitivity in many applications.

Several devices were developed in an attempt to overcome some of these problems. For example, U.S. Patent Application Publication No. 2004/0265440 to Morris et al. describes a sensor for detecting bacteria in a perishable food product. The sensor includes a gas-permeable material that contains a pH indicator carried by a housing for placement in a spaced relation to food product or packaging surfaces. The indicator detects a change in a gaseous bacterial metabolite concentration that is indicative of bacterial growth, wherein a pH change is affected by a presence of the metabolite. For instance, the pH indicator (e.g., a mixture of Bromothymol Blue and Methyl Orange) will undergo a visual color change from green to orange in the presence of an increased level of carbon dioxide gas, which diffuses through the pH indicator, reduces hydrogen ion concentration, and thus lowers the pH.

Unfortunately, such pH indicators are still problematic. For instance, the detection of a lowered pH only indicates that some bacteria might be present. The lowered pH does not, however, provide an indication regarding what type of bacteria is present. The need for selective identification of the type of bacteria is important for a variety of reasons. For example, some types of bacteria may not be considered harmful. In addition, the knowledge of which type of bacteria is present may also lead one to identify the particular source of contamination.

Ponder Jennifer et al., Detection and identification of pathogenic bacteria via colorimetric sensing array, Abstracts of Papers American Chemical Society, Vol. 227, No. Part 1, March 2004, page U88, describe that detection and identification of bacteria by sampling the headspace over the culture media was achieved using an array of metalloporyphyrins and other chemo-responsive dyes immobilized on a polyvinyliden difluoride membrane. Various volatile organic compounds are produced during the bacteria's metabolism; these allow for selective recognition of the different microorganisms. Escherichia coli, Pseudomonas aeruginosa, Staphylococcus aureus, and Moraxella catarrhalis grown on identical agars give unique color response for each bacterium.

As such, a need currently exists for a technique of rapidly and simply detecting the presence of microorganisms, and identifying the particular type of detected microorganism.

### Summary of the Invention

In accordance with one embodiment of the present invention, a method for detecting the presence of a microorganism is disclosed. The method comprises extracting a headspace gas produced by a culture of the microorganism, analyzing the extracted headspace gas and identifying a volatile compound associated with the microorganism culture, and selecting an indicator that is capable of undergoing a detectable color change in the presence of the identified volatile compound, applying the indicator to a surface of a substrate to form an indicator strip; and detecting the microorganism by observing a color change on the indicator strip.

In accordance with still another embodiment of the present invention, a substrate for detecting the presence of multiple microorganisms is disclosed. The substrate contains at least first and second indicator zones. A first indicator is contained within the first indicator zone in an amount effective to cause a detectable color change upon contact with a first volatile compound produced by a first microorganism. A second indicator is contained within the second indicator zone in an amount effective to cause a detectable color change upon contact with a second volatile compound produced by a second microorganism.

The first and the second indicators are selected from the group cousisting of potassium permanganate, dimethylaminocinnamaldehyde, 2,4-dinitrophenylhydrazine, and ammonium dichromate.

Other features and aspects of the present invention are discussed in greater detail below.

### Brief Description of the Drawings

A full and enabling disclosure of the present invention, including the best mode thereof, directed to one of ordinary skill in the art, is set forth more particularly in the remainder of the specification, which makes reference to the appended figure in which:
Fig. 1 is a schematic illustration of a solid phase microextraction "SPME" assembly that may be used in accordance with one embodiment of the present invention;
Fig. 2 is a total ion chromatogram obtained for *P. aeruginosa* in Example 1, in which the abundance of the volatile compounds are plotted versus retention time;
Fig. 3 is a total ion chromatogram obtained for *S. Aureus* in Example 2, in which the abundance of the volatile compounds are plotted versus retention time;
Fig. 4 is a total ion chromatogram obtained for *E. Coli* in Example 3, in which the abundance of the volatile compounds are plotted versus retention time;
Fig. 5 is a total ion chromatogram obtained for *C. Albicans* in Example 4, in which the abundance of the volatile compounds are plotted versus retention time;
Fig. 6 is a total ion chromatogram obtained for *Salmonella* in Example 5, in which the abundance of the volatile compounds are plotted versus retention time;
Fig. 7 represents an expansion of Fig. 6 in the region where some differences were observed.

### Detailed Description of Representative Embodiments

Reference now will be made in detail to various embodiments of the invention, one or more examples of which are set forth below. Each example is provided by way of explanation of the invention, not limitation of the invention. Features illustrated or described as part of one embodiment, may be used on another embodiment to yield a still further embodiment.

The present invention is directed to a technique for detecting the presence of microorganisms in a simple, rapid, and efficient manner. More specifically, the technique involves identifying one or more volatile compounds associated with a particular microorganism of interest. The volatile compounds may be identified, for instance, using solid phase microextraction in conjunction with gas chromatography / mass spectroscopy ("GC/MS") analysis methods. Once identified, an indicator may then be selected that is configured to undergo a detectable color change in the presence of the identified volatile compound(s). If desired, the indicator may be provided on a substrate to form an indicator strip for use in a wide variety of applications. In this manner, the presence of the microorganisms may be rapidly detected by simply observing a color change on the indicator strip.

Microorganisms, such as bacteria, yeast, fungi, mold, protozoa, viruses, etc., are classified into various groups depending on certain characteristics. For example, bacteria are generally classified based on their morphology, staining characteristics, environmental requirements, and metabolic characteristics, etc. Several medically significant bacterial groups include, for instance, gram negative rods (e.g., Entereobacteria); gram negative curved rods (e.g., vibious, *Heliobacter*, *Campylobacter*, etc.); gram negative cocci (e.g., *Neisseria*); gram positive rods (e.g., *Bacillus, Clostridium*, etc.); gram positive cocci (e.g., *Staphylococcus, Streptococcus*, etc.); obligate intracellular parasites (e.g., *Ricckettsia* and *Chlamydia*); acid fast rods (e.g., *Myobacterium, Nocardia*, etc.); spirochetes (e.g., *Treponema, Borellia*, etc.); and mycoplasmas (i.e., tiny bacteria that lack a cell wall). Particularly relevant bacteria include *E. coli* (gram negative rod), *Klebsiella pneumonia* (gram negative rod), *Streptococcus* (gram positive cocci), *Salmonella choleraesuis* (gram negative rod), *Staphyloccus aureus* (gram positive cocci), and *P. aeruginosa* (gram negative rod).

Under certain conditions, microorganisms grow and reproduce. The requirements for growth may include a supply of suitable nutrients, a source of energy (e.g., phototrophic or chemotrophic), water, an appropriate temperature, an appropriate pH, appropriate levels of oxygen (e.g., anaerobic or aerobic), etc. For instance, bacteria may utilize a wide range of compounds as nutrients, such as sugars and carbohydrates, amino acids, sterols, alcohols, hydrocarbons, methane, inorganic salts, and carbon dioxide. Growth proceeds most rapidly at the optimum growth temperature for particular bacteria (and decreases as temperature is raised or lowered from this optimum). For any bacteria, there is a minimum and maximum temperature beyond which growth is not supported. Thermophilic bacteria have an optimum growth temperature greater than 45°C, mesophilic bacteria have an optimum growth temperature between 15 and 45°C (e.g. human pathogenic bacteria), and psychrophilic bacteria have an optimum growth temperature of below 15°C.

Many types of microorganisms, including medically significant human pathogens, generate volatile compounds during growth and reproduction. The present inventors have discovered that one or more of these volatile compounds appear to be unique to a particular group, genus, species, and/or sub-species of microorganism. As such, the volatile compounds may be analyzed and identified to develop a detection technique specific for the identified volatile compound. The volatile compounds may be analyzed at or near a load that is the threshold safety level for the application of interest. For example, a bacteria load of 1 x 10³ colony forming units ("cfu") per milliliter of stock is accepted as the threshold safety level in food-based applications. Thus, in some embodiments, the analysis may occur at a load of at least about 1 x 10³ cfu per milliliter of stock. It should be understood, however, that the load selected for testing may or may not be the same as the accepted threshold level. That is, smaller loads may be tested so long as at least one or more volatile compounds are identifiable.

The volatile compounds generated by a microorganism may be analyzed in accordance with the present invention using a variety of different techniques. In some embodiments, extraction methods are employed, such as Soxhlet extraction, liquid-liquid extraction, accelerated solvent extraction, microwave-assisted solvent extraction, solid-phase extraction, supercritical fluid extraction, and so forth. One particularly desirable extraction technique is "solid phase microextraction" ("SPME"), which allows the performance of sample extraction and preconcentration in a single step. In SPME, the outer surface of a solid fused fiber is coated with a selective stationary phase. Thermally stable polymeric materials that allow fast solute diffusion are commonly used as the stationary phase. The extraction operation is carried out by dipping the coated fiber end into the headspace of the sample, and allowing the establishment of an equilibrium.

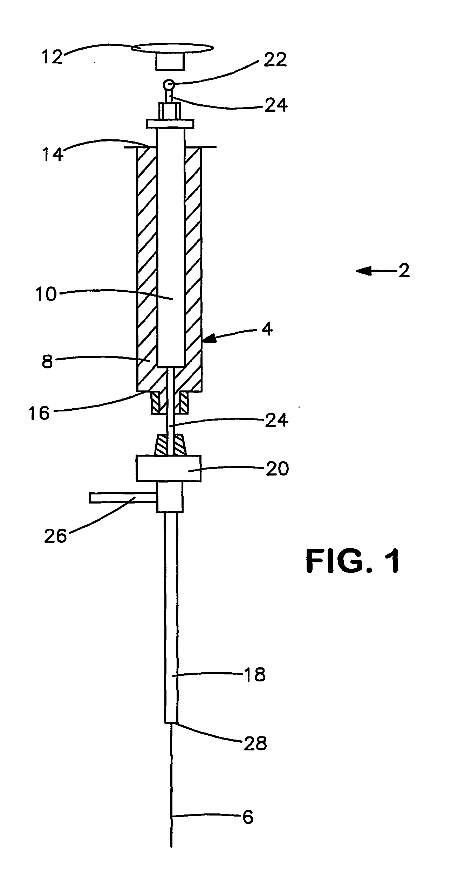

Referring to Fig. 1, for instance, one embodiment of a device 2 for carrying out solid phase microextraction is shown that uses a syringe 4. The syringe 4 is formed from a barrel 8 that contains a plunger 10 slidable within the barrel 8. The plunger 10 has a handle 12 extending from one end 14 of the barrel 8. A needle 18 is connected to an opposite end 16 of the barrel 8 by a connector 20. The device 2 also includes a fiber 6, which is a solid thread-like material that extends from the needle 18 through the barrel 8 and out the end 14. An end of the fiber 6 (not shown) located adjacent to the handle 12 has retention means 22 located thereon so that the fiber will move longitudinally as the plunger 10 slides within the barrel 8. The retention means may simply be a drop of epoxy placed on the end of the fiber 6 near the handle 8. The fiber 6 is partially enclosed in a metal sleeve 24 that surrounds the portion of the fiber 6 within the plunger 10, the barrel 8 and part of the needle 18. One purpose of the metal sleeve 24 is to protect the fiber 6 from damage and to ensure a good seal during operation of the device. Extending from the connector 20 is an optional inlet 26 that allows alternate access to the fiber 6. For example, when the fiber 6 is contained within the needle 18, fluid may contact the fiber 6 by entering the inlet 26 and exiting from a free end 28 of the needle 18. The inlet 26 may also be used to contact the fiber 6 with an activating solvent.

To perform solid phase microextraction and subsequent analysis, a user may simply depress the plunger 10 and exposed fiber 6 into a container, bottle, dish, agar plates, or any other sample holder that includes the relevant microorganism culture. For example, the fiber may be fused silica coated with a liquid phase (polydimethylsiloxane, styrene divinylbenzene porous polymer, polyethylene glycol, carbon molecular sieve adsorbent, combinations thereof, and so forth). During the culture period, the microorganisms grow and produce the relevant volatile compounds. As such, the headspace of the sample holder becomes filled with the volatile compounds. After a sufficient period of time (e.g., from about 2 to about 30 minutes), the headspace components are adsorbed onto the fiber 6. Thereafter, the plunger 10 is moved to the withdrawn position to draw the fiber 6 into the needle 8, and the needle 8 is then removed from the sample holder. The adsorbed headspace components are then desorbed from the fiber liquid phase by heating for subsequent analysis. One SPME assembly suitable for use in the present invention is commercially available from Sigma-Aldrich, Inc. of St. Louis, Missouri under the name "Supelco." The "Supelco" system utilizes a manual fiber holder (catalog no. 57330-U) and a StableFlex™ fiber coated with 85 µm-Carboxen^{™} / polydimethylsilicone (catalog no. 57334-U). Such fibers are recommended for gases and low molecular weight compounds. In addition, various other extraction techniques may also be employed in the present invention, such as described in U.S. Patent Nos. 5,691,206 to Pawliszyn; 6,537,827 to Pawliszyn; 6,759,126 to Malik, et al.; and 6,780,314 to Jinno. et al..

Extraction is normally followed by chromatographic analysis in which the extracted compounds are desorbed in an injection port for introduction into a chromatographic column. For example, in one embodiment, a gas chromatograph ("GC") is employed for use in the present invention. A gas chromatograph ("GC") is an analytical instrument that takes a gaseous sample, and separates the sample into individual compounds, allowing the identification and quantification of those compounds. A typical gas chromatograph includes an injector that converts sample components into gases and moves the gases onto the head of the separation column in a narrow band and a separation column (e.g., long, coiled tube) that separates the sample mixture into its individual components as they are swept through the column by an inert carrier gas. Separation is based on differential interactions between the components and an immobilized liquid or solid material within the column. For example, when employed in the present invention, the extracted headspace gas is received in an inlet of the gas chromatograph. The gas then moves through a column that separates the molecules. The different sample components are retained for different lengths of time within the column, and arrive at characteristic retention times. These "retention times" may be used to identify the particular sample components, and are a function of the type and amount of sorbtive material in the column, the column length and diameter, the carrier gas type and flow rate, and of the column temperature.

The gas chromatograph is generally controllably heated or cooled to help obtain reproducible retention times. For example, an oven may be employed that heats a polyimide or metal clad fused silica tube coated with a variety of coatings (e.g., polysiloxane based coatings). The oven may use a resistive heating element and a fan that circulates heated air in the oven. The column may likewise be cooled by opening vents in the oven, turning off the resistive heating element, and using forced air cooling of the column with ambient air or cryogenic coolant, such as liquid carbon dioxide or liquid nitrogen. Alternatively, a metal sheath may be used to heat a capillary GC column. In this case, the column is threaded into the metal sheath, and then the sheath is resistively heated during the chromatographic process.

If desired, GC analysis may also be interfaced with other identification techniques, including mass spectroscopy ("MS"), to achieve more accurate results. Mass spectroscopy is generally an analytical methodology used for quantitative and qualitative chemical analysis of materials and mixtures of materials. In mass spectroscopy, the sample (i.e., separated by the GC process) is broken into electrically charged particles of its constituent parts in an ion source. Once produced, the particles are further separated by the spectrometer based on their respective mass-to-charge ratios. The ions are then detected and a mass spectrum of the material is produced. The mass spectrum is analogous to a fingerprint of the sample material being analyzed in that it provides information about the ion intensities of the eluting molecules. In particular, mass spectroscopy may be used to determine the molecular weights of molecules and molecular fragments of a sample. A mass spectrometer generally contains an ionization source that produces ions from the sample. For example, one type of ionization source that may be employed is an electron ionization (EI) chamber. The mass spectrometer also typically contains at least one analyzer or filter that separates the ions according to their mass-to-charge ratio (m/z), and a detector that measures the abundance of the ions. The detector, in turn, provides an output signal to a data processing system that produces a mass spectrum of the sample.

The GC and MS components of a GC/MS system may be separate or integrated. For example, one integrated GC/MS system suitable for use in the present invention is commercially available from Agilent Technologies, Inc. of Loveland, Colorado under the name "5973N." Various other gas chromatograph and/or mass spectroscopy systems are also described in 5,846,292 to Overton; 6,691,053 to Quimby, et al.; 6,607,580 to Hustings, et al.; 6,646,256 to Gourley, et al.; and 6,849,847 to Bai, et al.

Regardless of the particular identification technique employed, it is normally desired to identify a volatile compound that is unique to a particular genus, species, and/or sub-species of microorganism. In this manner, an indicator may be selected that is specific for the identified volatile compound. In practice, however, it may be difficult to readily determine whether an identified volatile compound is truly unique. Thus, unique volatile compounds may be identified based on the types of microorganisms that the indicator is likely to encounter during use. For example, some types of bacteria considered relevant in food-based applications include *E. coli, S. choleraesuis, S. aureus*, and *P. aeruginosa.* Likewise, if the indicator is intended for a wide range of uses, a larger number of bacteria types may be tested. In still other cases, it may not even be necessary or desired to identify a volatile compound that is unique to a particular type of microorganism. For example, one or more volatile compounds may be identified for one type of microorganism that are also produced by another type of microorganism. In such cases, the indicator will still identify the presence of one or more of the microorganism types.

Generally, the indicator is capable of readily signifying the presence of an identified volatile compound. In one embodiment, the indicator is a dye that exhibits a color change that is detectable, either visually or through instrumentation, upon contact with the volatile compound. For example, prior to contact with the volatile compound, the indicator dye may be colorless or it may possess a certain color. However, after contacting the volatile compound, the dye exhibits a change in color that is different than its initial color. That is, the dye may change from a first color to a second color, from no color to a color, or from a color to no color. Although not required, the detectable color change may result from the addition of a functional group (e.g., OH, NH₂, etc.) to the dye molecule that induces either a shift of the absorption maxima towards the red end of the spectrum ("bathochromic shift") or towards the blue end of the spectrum ("hypsochromic shift"). The type of absorption shift depends on the nature of the dye molecule and on whether the functional group functions as an electron acceptor (oxidizing agent), in which a hypsochromic shift results, or whether the functional group functions as an electron donor (reducing agent), in which a bathochromic shift results. Regardless, the absorption shift may provide the detectable color difference.

Any of a variety of known indicators may be utilized in the present invention. For example, one such indicator is 4-dimethylaminocinnamaldehyde, which is an ethylenically unsaturated amine base having the following structure: 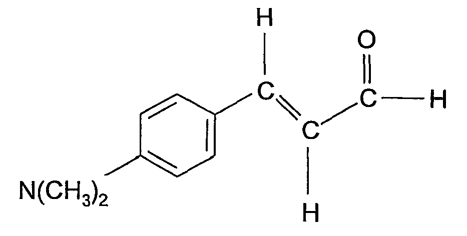 4-dimethylaminocinnamaldehyde is capable of undergoing a color change in the presence of indole, which generally has the following structure:

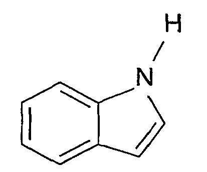

Another suitable indicator is potassium permanganate, which has the following structure: 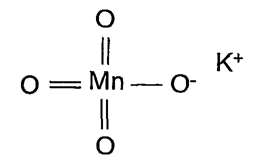 Potassium permanganate is a strong oxidizer and is capable of undergoing a color change in the presence of readily oxidizable compounds, such as alcohols, aldehydes, unsaturated hydrocarbons, and so forth. One example of such a readily oxidzable compound is methyl 2-methyl-2-butenoate, which has the following structure:

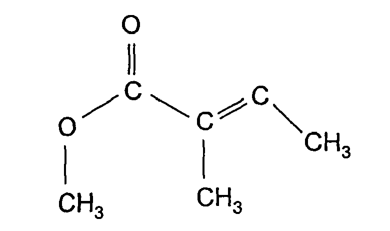

Another example of a compound that is capable of initiating a color change in potassium permanganate is 2,5-dimethylpyrazine, which has the following structure:

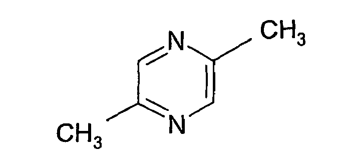

Still another suitable indicator is ammonium dichromate, which has the following structure: 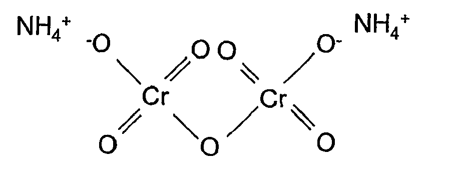 Ammonium dichromate is also a strong oxidizer and is capable of undergoing a color change in the presence of alcohols, such as iso-amyl alcohol, which has the formula, (CH₃)₂CHCH₂CH₂OH.

Further, 2,4-dinitrophenylhydrazine ("DPNH") is also a suitable indicator for compounds having a carbon-oxygen double bond (e.g., aldehydes and ketones), and has the following structure: 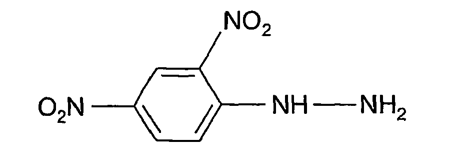 For example, DPNH (also known as "Brady's reagent") is capable of undergoing a color change in the presence of 2-acetyl thiazole, which has the following structure:

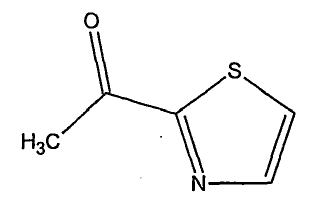

Besides the indicators mentioned above, some other common indicators and their associated target compounds are set forth below.

| Indicator | Target Compound |
|---|---|
| ammonia | tetracyclines |
| ammonium cerium(IV)nitrate | polyalcohols |
| aniline/phosphoric acid | sugars |
| p-anisaldehyde | reducing sugars |
| p-anisidine phthalate | reducing sugars |
| anthrone | ketoses |
| bismuth chloride | sterols |
| bromocresol green | organic and inorganic acids |
| bromocresol purple | dicarboxylic acids, halogen ions |
| carmine | polysaccharides |
| chromosulfuric acid | organic compounds |
| cobalt(II)chloride | organic phosphate esters |
| cobalt(II)thiocyanate | alkaloids, amines |
| alpha-cyclodextrin | straight-chain lipids |
| o-dianisidine | aldehydes, ketones |
| 2,6-dibromoquinone chlorimide | phenols |
| 2',7'-dichlorofluorescein | saturated and unsaturated lipids |
| 2,6-dichlorophenolindophenol | organic acids, keto acids |
| dicobalt octacarbonyl | acetylene compounds |
| diethyl malonate | 3,5-dinitrobenzoic acid esters |
| 3,5-dinitrobenzoic acid | reducing sugars |
| 2,4-dinitrofluorobenzene | amino acids |
| 3,5-dinitrosalicylic acid | reducing sugars |
| diphenylamine | glycolipids |
| diphenylcarbazone | cations |
| 4,4'-dithiodianils | thiols |
| ethylenediamine | catechol amines |
| Fast blue salt B | phenols, coupling amines |
| fluorescein | lipids |
| glyoxalbis(2-hydroxyanil) | cations |
| hydrazine sulfate | piperonal, vanillin, ethyl vanillin |
| hydrochloric acid | glycals |
| hydrogen peroxide | aromatic acids |
| iron(II)thiocyanate | peroxides |
| lead(IV)acetate | 1,2-diol groups |
| magnesium acetate | anthraquinone glycosides |
| methylunmbelliferone | heterocyclic compounds |
| 1-naphthol/hypobromite | guanidine derivatives |
| ninhydrin | amino acids, amines, amino-sugars, |
| palladium(II)chloride | thiophosphate esters |
| phenol/sulfuric acid | sugars |
| m-phenylenediamine | reducing sugars |
| phenylfluorone | germanium |
| phenylhydrazine | dehydroascorbic acid |
| quinalizarin | cations |
| p-quinone | ethanolamine |
| rhodanine | carotenoid aldehydes |
| silver nitrate | phenols |
| sodium meta-periodate | hydroxyamino acids, serine, threonine |
| sodium nitroprusside | compounds with sulfhydryl group |
| tetracyanoethylene | aromatic hydrocarbons, phenols |
| tetrazolium blue | reducing compounds |
| thiobarbituric acid | sorbic acid |
| thymol blue | dimethylamino acids |
| tin(IV) chloride | triterpenes, phenols, polyphenols |
| toluidine blue | acidic polysaccharides |
| xanthydrol | tryptophan, indole derivatives |

The selected indicator is generally used in an amount effective to achieve a detectable color change in the presence of a certain microorganism. The ability of the indicator to achieve the desired color change may be enhanced by increasing the contact area between the indicator and the gas generated by the microorganism. In turn, this may reduce the amount of indicator needed to achieve the desired color change. One technique for increasing surface area in such a manner is to apply the indicator to a substrate. When employed, one or more indicators may be applied to the substrate to form an indicator strip that is configured to detect the presence of one or multiple types of microorganisms. For example, in one embodiment, the indicator strip may be designed to detect the presence of *E. coli, S. choleraesuis, S. aureus,* and *P. aeruginosa.* This may be accomplished using a single indicator or multiple indicators (e.g., four). The substrate may also serve other purposes, such as water absorption, packaging, etc.

Any of a variety of different substrates may be incorporated with the indicator in accordance with the present invention. For instance, nonwoven fabrics, woven fabrics, cotton, knit fabrics, wet-strength papers, films, foams, etc., may be applied with the indicator. When utilized, nonwoven fabrics may include, but are not limited to, spunbonded webs (apertured or non-apertured), meltblown webs, bonded carded webs, air-laid webs, coform webs, hydraulically entangled webs, and so forth. A wide variety of thermoplastic materials may be used to construct nonwoven webs, including without limitation polyamides, polyesters, polyolefins, copolymers of ethylene and propylene, copolymers of ethylene or propylene with a C₄-C₂₀ alpha-olefin, terpolymers of ethylene with propylene and a C₄-C₂₀ alpha-olefin, ethylene vinyl acetate copolymers, propylene vinyl acetate copolymers, styrene-poly(ethylene-alpha-olefin) elastomers, polyurethanes, A-B block copolymers where A is formed of poly(vinyl arene) moieties such as polystyrene and B is an elastomeric midblock such as a conjugated diene or lower alkene, polyethers, polyether esters, polyacrylates, ethylene alkyl acrylates, polyisobutylene, poly-1-butene, copolymers of poly-1-butene including ethylene-1-butene copolymers, polybutadiene, isobutylene-isoprene copolymers, and combinations of any of the foregoing.

Another type of suitable nonwoven web is a coform material, which is typically a blend of cellulose fibers and meltblown fibers. The term "coform" generally refers to composite materials comprising a mixture or stabilized matrix of thermoplastic fibers and a second non-thermoplastic material. As an example, coform materials may be made by a process in which at least one meltblown die head is arranged near a chute through which other materials are added to the web while it is forming. Such other materials may include, but are not limited to, fibrous organic materials such as woody or non-woody pulp such as cotton, rayon, recycled paper, pulp fluff and also superabsorbent particles, inorganic absorbent materials, treated polymeric staple fibers and so forth. Some examples of such coform materials are disclosed in U.S. Patent Nos. 4,100.324 to Anderson, et al.; 5,284,703 to Everhart, et al.; and 5,350,624 to Georger, et al.

The indicator may also be utilized in a paper product containing one or more paper webs, such as facial tissue, bath tissue, paper towels, napkins, and so forth. The paper product may be single-ply in which the web forming the product includes a single layer or is stratified (i.e., has multiple layers), or multi-ply, in which the webs forming the product may themselves be either single or multilayered. Any of a variety of materials may also be used to form a paper web. For example, the paper web may include fibers formed by a variety of pulping processes, such as kraft pulp, sulfite pulp, thermomechanical pulp, etc.

In addition, the substrate may also contain a film. A variety of materials may be utilized to form the films. For instance, some suitable thermoplastic polymers used in the fabrication of films may include, but are not limited to, polyolefins (e.g., polyethylene, polypropylene, etc.), including homopolymers, copolymers, terpolymers and blends thereof; ethylene vinyl acetate; ethylene ethyl acrylate; ethylene acrylic acid: ethylene methyl acrylate; ethylene normal butyl acrylate; polyurethane; poly(ether-ester); poly(amid-ether) block copolymers; and so forth.

Whether containing films, nonwoven webs, etc., the permeability of a substrate utilized in the present invention may also be varied for a particular application. For example, in some embodiments, the substrate may be permeable to liquids. Such substrates, for example, may be useful in various types of fluid absorption and filtration applications. In other embodiments, the substrate may impermeable to liquids, gases, and water vapor, such as films formed from polypropylene or polyethylene. In still other embodiments, the substrate may be impermeable to liquids, but permeable to gases and water vapor (i.e., breathable). The "breathability" of a material is measured in terms of water vapor transmission rate (WVTR), with higher values representing a more vapor-pervious material and lower values representing a less vapor-pervious material. Breathable materials may, for example, have a water vapor transmission rate (WVTR) of at least about 100 grams per square meter per 24 hours (g/m²/24 hours), in some embodiments from about 500 to about 20,000 g/m²/24 hours, and in some embodiments, from about 1,000 to about 15,000 g/m²/24 hours. The breathable material may generally be formed from a variety of materials as is well known in the art. For example, the breathable material may contain a breathable film, such as a microporous or monolithic film.

The indicator may be applied to a substrate using any of a variety of well-known application techniques. Suitable application techniques include printing, dipping, spraying, melt extruding, solvent coating, powder coating, and so forth. The indicator may be incorporated within the matrix of the substrate and/or contained on the surface thereof. For example, in one embodiment, the indicator is coated onto one or more surfaces of the substrate. In one particular embodiment, an indicator coating is printed onto a substrate using printing techniques, such as flexographic printing, gravure printing, screen printing, or ink jet printing. Various examples of such printing techniques are described in U.S. Patent No. 5,853,859 to Levy, et al. and U.S. Patent Application Publication No. 2004/0120904 to Lye, et al.

If desired, the indicator may be applied to the substrate in one or more distinct zones so that a user may better determine the presence of a particular microorganism. For example, two or more distinct indicator zones (e.g., lines, dots, etc.) may be used to detect the presence of two or more microorganisms. In one particular embodiment, four different indicator zones are used to detect the presence of *E. coli,* S. *choleraesuis, S. aureus,* and *P. aeruginosa.* In this manner, a user may simply observe the different zones to determine which microorganisms are present. Although the indicator zones may generally be applied to any surface of the substrate, they are typically present on at least a surface that is capable of contacting the volatile compounds produced by the microorganisms during use.

The amount of the indicator present on the substrate may vary depending on the nature of the substrate and its intended application, the nature of the indicator and volatile compounds, and so forth. For example, lower add-on levels may provide optimum functionality of the substrate, while higher add-on levels may provide optimum detection sensitivity. Nevertheless, the indicator will generally range from about 0.001 wt.% to about 10 wt.%, in some embodiments from about 0.01 wt.% to about 5 wt.%, and in some embodiments, from about 0.05 wt.% to about 2 wt.% of the substrate. Likewise, the percent coverage of the indicator on the surface of a substrate may be selectively varied. Typically, the percent coverage is less than 100%, in some embodiments less than about 90%, and in some embodiments, from about 5% to about 50% of the area of a given surface.

In some cases, high-surface area particles may also be employed to increase the effective surface of the substrate, thereby improving contact between the indicator and volatile compounds. Generally, the high-surface area particles have a surface area of from about 50 square meters per gram (m²/g) to about 1000 m²/g, in some embodiments from about 100 m²/g to about 600 m²/g, and in some embodiments, from about 180 m²/g to about 240 m²/g. Surface area may be determined by the physical gas adsorption (B.E.T.) method of Bruanauer, Emmet, and Teller, Journal of American Chemical Society, Vol. 60, 1938, p. 309, with nitrogen as the adsorption gas. The high-surface area particles may also possess various forms, shapes, and sizes depending upon the desired result. For instance, the particles may be in the shape of a sphere, crystal, rod, disk, tube, string, etc. The average size of the particles is generally less than about 100 nanometers, in some embodiments from about 1 to about 50 nanometers, in some embodiments from about 2 to about 50 nanometers, and in some embodiments, from about 4 to about 20 nanometers. As used herein, the average size of a particle refers to its average length, width, height, and/or diameter. In addition, the particles may also be relatively nonporous or solid. That is, the particles may have a pore volume that is less than about 0.5 milliliters per gram (ml/g), in some embodiments less than about 0.4 milliliters per gram, in some embodiments less than about 0.3 ml/g, and in some embodiments, from about 0.2 ml/g to about 0.3 ml/g.

The high-surface area particles may be formed from a variety of materials, including, but not limited to, silica, alumina, zirconia, magnesium oxide, titanium dioxide, iron oxide, zinc oxide, copper oxide, organic compounds such as polystyrene, and combinations thereof. For example, alumina nanoparticles may be used. Some suitable alumina nanoparticles are described in U.S. Patent No. 5,407,600 to Ando, et al.. Further, examples of commercially available alumina nanoparticles include, for instance, Aluminasol 100, Aluminasol 200, and Aluminasol 520, which are available from Nissan Chemical Industries Ltd. Alternatively, silica nanoparticles may be utilized, such as Snowtex-C, Snowtex-O. Snowtex-PS, and Snowtex-OXS, which are also available from Nissan Chemical. Snowtex-OXS particles, for instance, have a particle size of from 4 to 6 nanometers, and may be ground into a powder having a surface area of approximately 509 square meters per gram. Also, alumina-coated silica particles may be used, such as Snowtex-AK available from Nissan Chemical.

High-surface area particles, such as referenced above, may possess units that may or may not be joined together. Whether or not such units are joined generally depends on the conditions of polymerization. For instance, when forming silica nanoparticles, the acidification of a silicate solution may yield Si(OH)₄. If the pH of this solution is reduced below 7 or if a salt is added, then the units may tend to fuse together in chains and form a "silica gel." On the other hand, if the pH is kept at a neutral pH or above 7, the units may tend to separate and gradually grow to form a "silica sol." Such colloidal silica nanoparticles may generally be formed according to any of a variety of techniques well known in the art, such as dialysis, electrodialysis, peptization, acid neutralization, and ion exchange. Some examples of such techniques are described, for instance, in U.S. Patent Nos. 5,100,581 to Watanabe, et al.; 5,196,177 to Watanabe, et al.; 5,230,953 to Tsugeno, et al. and 5,985,229 to Yamada et al.

In one particular embodiment, a silica nanoparticle sol is formed using an ion-exchange technique. For exemplary purposes only, one such ion-exchange technique will now be described in more detail. Initially, an alkali metal silicate is provided that has a molar ratio of silicon (SiO₂) to alkali metals (M₂O) of from about 0.5 to about 4.5. For instance, sodium water glass may be utilized that has a molar ratio of from about 2 to about 4. An aqueous solution of the alkali metal silicate is obtained by dissolving it in water at a concentration of, for instance, from about 2 wt.% to about 6 wt.%. The alkali metal silicate-containing aqueous solution may then be contacted with one or more ion-exchange resins. For instance, the solution may first be contacted with a strong-acid to ion-exchange all the metal ions in the aqueous solution. Examples of such strong acids include, but are not limited to, hydrochloric acid, nitric acid, sulfuric acid, and so forth. The contact may be accomplished by passing the aqueous solution through a column filled with the strong acid at a temperature of from about 0°C to about 60°C, and in some embodiments, from about 5°C to about 50°C. After passing through the column, the resulting silicic acid-containing aqueous solution may have a pH value of from about 2 to about 4. If desired, another strong acid may be added to the silicic acid-containing aqueous solution to convert the impurity metal components into dissociated ions. This additional strong acid may decrease the pH value of the resulting solution to less than about 2, and in some embodiments, from about 0.5 to about 1.8.

The metal ions and the anions from the strong acid may be removed from the solution by consecutive application of a strong acid (i.e., cation-exchange resin) and strong base (anion-exchange resin). Examples of suitable strong bases include, but are not limited to, sodium hydroxide, potassium hydroxide, and so forth. As a result of this consecutive application, the silicic acid-containing aqueous solution may have a pH value of from about 2 to about 5. This acidic aqueous solution may then be contacted with one or more additional strong bases to stabilize the solution at a pH value of from about 7 to about 9.

The stabilized silicic acid-containing aqueous solution is then fed to a container in which the liquid temperature is maintained at from about 70°C to about 100°C. This process results in an increase in concentration of the silica to from about 30 wt.% to about 50 wt.%. The stable aqueous silica sol may then be consecutively contacted with a strong acid and strong base, such as described above, so that the resulting aqueous silica sol is substantially free from polyvalent metal oxides, other than silica. Finally, ammonia may be added to the aqueous sol to further increase its pH value to from about 8 to about 10.5, thereby forming a stable aqueous silica sol having a silica concentration of from about 30 wt.% to about 50 wt.%, a mean particle size of from about 10 to about 30 nanometers, and that is substantially free from any polyvalent metal oxides, other than silica.

The amount of the particles may generally vary depending on the nature of the substrate and its intended application. In some embodiments, for example, the dry, solids add-on level is from about 0.001 % to about 20%, in some embodiments from about 0.01% to about 10%, and in some embodiments, from about 0.1 % to about 4%. The "solids add-on level" is determined by subtracting the weight of the untreated substrate from the weight of the treated substrate (after drying), dividing this calculated weight by the weight of the untreated substrate, and then multiplying by 100%. Lower add-on levels may provide optimum functionality of the substrate, while higher add-on levels may provide optimum contact between the volatile compounds and the indicator.

In accordance with the present invention, a color change in the indicator strip serves as a simple and quick signal for the presence of a microorganism. This may be useful in a variety of applications, including in the evaluation of food safety, infection, odor, etc. For instance, the indicator strip may be used as or in conjunction with a wound care dressing; food packaging; refrigerators; toys; food preparation areas; hospital areas; bathroom areas; telephones; computer equipment; feminine pads; diapers; and so forth. If desired, the indicator strip may also include an adhesive (e.g., pressure-sensitive adhesive, melt adhesive, etc.) for adhering the strip to the desired surface.

The present invention may be better understood with reference to the following examples.

### EXAMPLE 1

The ability to identify one or more volatile compounds associated with a particular type of bacteria was demonstrated. In this particular example, *P. aeruginosa* (ATCC #9027) was tested. A suspension of *P. aeruginosa* was prepared by diluting a 1 x 10⁸ colony forming unit (cfu) per milliliter (ml) stock of the microorganism with a tryticase soy broth ("TSB") solution to a concentration of 1 x 10⁵ cfu/ml. One milliliter of the suspension was applied to Dextrose Sabouraud agar plates, and allowed to grow at 35°C for 4 hours. A sample was prepared by placing the *P. aeruginosa* suspension into 250-milliliter septa-jar and sealing it with an aluminum foil-lined Teflon/silicone cap. For control purposes, a nutrient blank was also prepared.

The test and control samples were exposed to a 85-micrometer Carboxen^{™}, / polydimethylsilicone "Solid Phase Microextraction" (SPME) assembly for about 30 minutes to collect the volatiles for analysis. (Supelco catalog No. 57330-U manual fiber holder and 57334-U 85 µm Carboxen^{™} / polydimethylsilicone on a StableFlex fiber, which are recommended for gases and low molecular weight compounds). Gas chromatography and mass spectrometery (GC/MS) analysis was conducted using a system available from Agilent Technologies, Inc. of Loveland. Colorado under the series name "5973N." Helium was used as the carrier gas (injection port pressure: 1.89 × 10⁵ Pa (12.7 psig); supply line pressure is at 5.15 × 10⁵ Pa (60 psig)). A DB-5MS column was used that had a length of 60 meters and an internal diameter of 0.25 millimeters. Such a column is available from J&W Scientific, Inc. of Folsom, California. The oven and operating parameters used for the GC/MS system are shown below in Tables 1 and 2:

**Table 1: Oven Parameters for the GC/MS System**

| Level | Rate (°C/min.) | Final Temp. (°C) | Final Time (min) |
|---|---|---|---|
| Initial | | -20 | 4.00 |
| 1 | 10 | 200 | 0.00 |
| 2 | 15 | 250 | 0.00 |

**Table 2: Operating Parameters for the GC/MS System**

| Parameter | Value | |
|---|---|---|
| Carrier Gas | Helium at constant flow (1.9 ml/min) | |
| Injector | Split ratio | 5:1 |
| | Temperature | 225°C |
| Detector | Source Temp. | 230°C |
| | Quad Temp. | 150°C |
| | Interface | 260°C |
| | EM | 1906 v |
| | HED | on |
| | Scan Range | 12 - 350 Dalton |
| | Threshold | 100 |
| | A/D Samples | 8 |
| Solvent Delay | 0.0 minutes | |

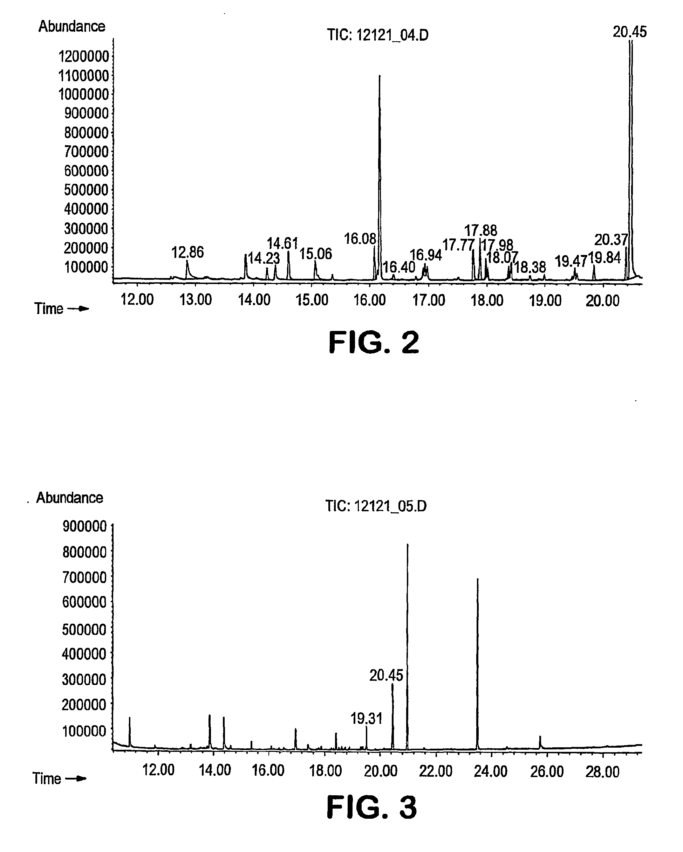

The SPME extracts were thermally desorbed and the isolates were analyzed by GC/MS. The acquired total ion chromatogram for *P. aeruginosa* is set forth in Fig. 2. The peaks of the spectrum were matched to a corresponding compound using the mass-to-charge ratios in the spectrum and their relative abundance. The results of the spectral analysis are shown below in Table 3.

**Table 3: Spectral Analysis for P. aeruginosa**

| **Time** | **CAS No.** | **Compound** |
|---|---|---|
| 12.85 | 1534-08-3 | ethanoic acid (S-methyl ester) |
| 14.22 | 97-62-1 | 2-methyl-propanoic acid (ethyl ester) |
| 14.60 | 556-24-1 | 3-methyl-butanoic acid (methyl ester) |
| 15.06 | 1679-08-9 | 2,2-dimethyl-1-propanethiol |
| 16.08 | 7452-79-1 | 2-methylbutanoic acid (ethyl ester) |
| 16.16 | 108-64-5 | 3-methylbutanoic acid (ethyl ester) |
| 16.40 | 6622-76-0 | methyl tiglate (2-methyl-2-butenoic acid, methyl ester) |
| 16.94 | 32665-23-9 | 3-methyl butanoic acid (isopropyl ester) |
| 17.77 | 5837-78-5 | ethyl tiglate (2-ethyl-2-butenoic acid, ethyl ester) |
| 17.88 | 23747-45-7 | S-methyl-3-methyl butanethioate |
| 17.98 | 141-06-0 | propyl pentanoate |
| 18.01 | 13678-59-6 | 2-methyl-5-(methylthio) furan |
| 18.38 | 1733-25-1 | isopropyl tiglate (2-isopropyl-2-butenoic acid, isopropyl ester) |
| 19.47 | 61692-83-9 | propyl tiglate (2-propyl-2-butenoic acid, propyl ester) |
| 19.83 | - | - |
| 20.37 | - | - |
| 20.44 | 821-95-4 | 1-undecene |

As indicated, several volatile compounds were identified as being associated with *P. aeruginosa,* including alkyl esters, sulfur-containing compounds, and an alkene.

### EXAMPLE 2

The procedure of Example 1 was utilized to identify volatile compounds produced by *S. aureus* (ATCC #6538). The acquired total ion chromatogram for *S. aureus* is set forth in Fig. 3. The peaks of the spectrum were matched to a corresponding compound using the mass-to-charge ratios in the spectrum and their relative abundance. Only two compounds exhibited a peak substantially greater in concentration than the nutrient blank. These two compounds are identified below in Table 4.

**Table 4: Spectral Analysis for S. aureus**

| Time | CAS No. | Compound |
|---|---|---|
| 19.31 | 24295-03-2 | 2-acetylthiazole |
| 20.44 | 821-95-4 | 1-undecene |

As indicated, the volatile compounds identified as being associated with *S. Aureus* included a thiazole and alkene.

### EXAMPLE 3

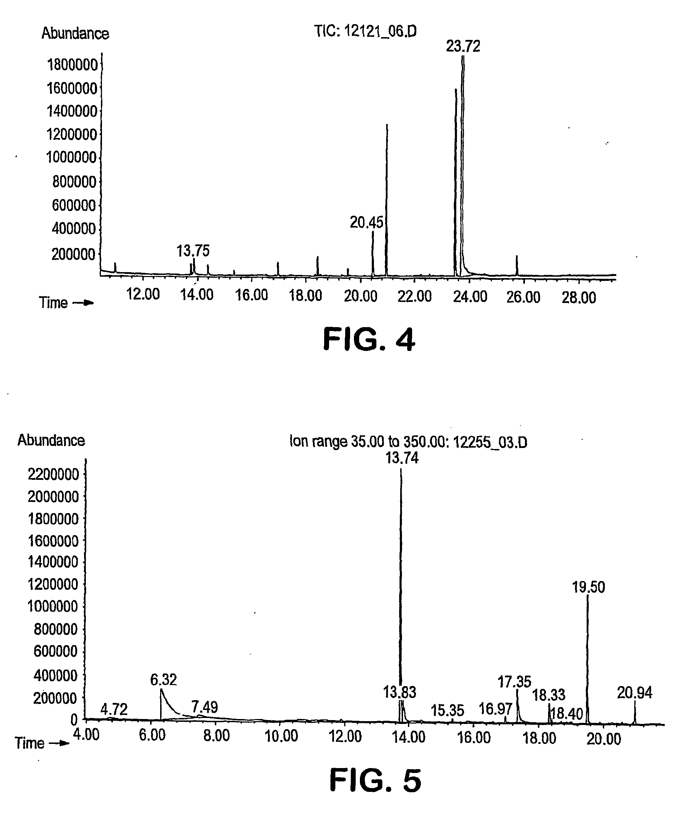

The procedure of Example 1 was utilized to identify volatile compounds produced by *E. coli* (ATCC #8739). The acquired total ion chromatogram for *E. coli* is set forth in Fig. 4. The peaks of the spectrum were matched to a corresponding compound using the mass-to-charge ratios in the spectrum and their relative abundance. Only three compounds exhibited a peak substantially greater in concentration than the nutrient blank. These three compounds are identified below in Table 5.

**Table 5: Spectral Analysis for E. coli**

| **Time** | **CAS No.** | **Compound** |
|---|---|---|
| 13.75 | 123-51-3 | 3-methyl-1-butanol |
| 20.44 | 821-95-4 | 1-undecene |
| 23.72 | 120-72-9 | indole |

As indicated, the volatile compounds identified as being associated with *E. coli* included an alcohol, alkene, and fused heterocyclic compound.

### EXAMPLE 4

The procedure of Example 1 was utilized to identify volatile compounds produced by *C. albicans* (ATCC# 10231). The acquired total ion chromatogram for *C. albicans* is set forth in Fig. 5. The peaks of the spectrum were matched to a corresponding compound using the mass-to-charge ratios in the spectrum and their relative abundance. The compounds are identified below in Table 6.

**Table 6: Spectral Analysis for C. albicans**

| **Time** | **Area** | **% of Total Area** | **Compound** |
|---|---|---|---|
| 4.8 | 2,363,467 | 1.9% | Acetaldehyde |
| 6.3 | 53,364,422 | 43.2% | Ethanol |
| 7.5 | 4,404,313 | 3.6% | Acetone |
| 13.7 | 32,245,674 | 26.1% | Isoamyl Alcohol |
| 13.8 | 3,449,096 | 2.8% | 2-methyl-1-butanol |
| 13.9 | 2,663,331 | 2.2% | Dimethyl disulfide |
| 17.0 | 1,016,473 | 0.8% | Styrene |
| 17.3 | 6,199,544 | 5.0% | 2,5-dimethyl pyrazine |
| 18.3 | 2,572,152 | 2.1% | Benzaldehyde |
| 19.5 | 15,040,131 | 12.2% | Limonene |
| 20.9 | 336,969 | 0.3% | Phenethyl alcohol |

As indicated, the volatile compounds identified as being associated with *C. albicans* included aldehydes, alcohols, heterocyclic compounds, and sulfur-containing compounds.

### EXAMPLE 5

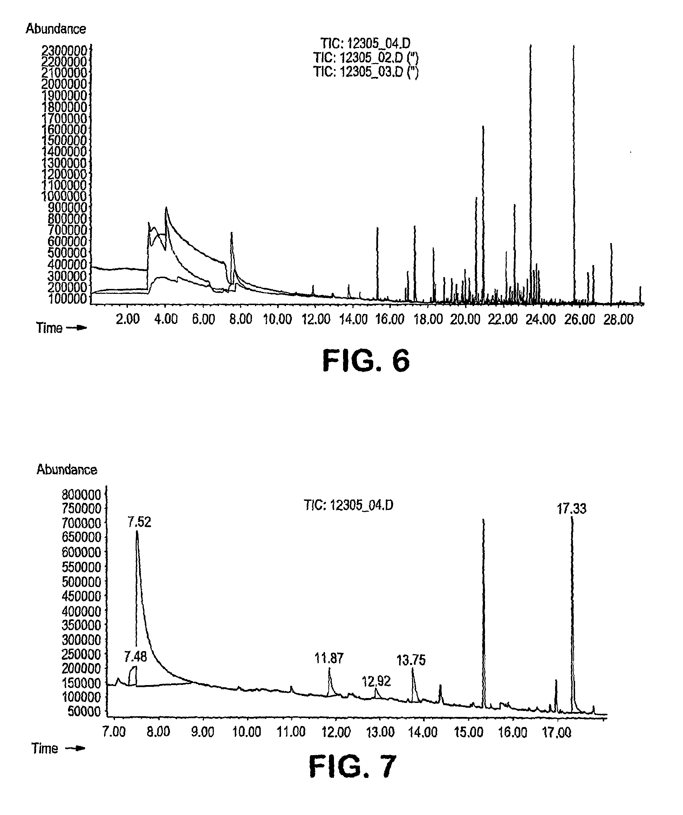

The procedure of Example 1 was utilized to identify volatile compounds produced by *Salmonella choleraesuis*. The acquired total ion chromatogram for *S. choleraesuis* is set forth in Figs. 6-7. The peaks of the spectrum were matched to a corresponding compound using the mass-to-charge ratios in the spectrum and their relative abundance. The compounds are identified below in Table 7.

**Table 7: Spectral Analysis for S. choleraesuis**

| **Time** | **Area** | **% of Total Area** | **Compound** |
|---|---|---|---|
| 7.48 | 4,997,820 | 4.6% | Ethanol |
| 7.52 | 81,647,036 | 75.2% | Isopropanol |
| 11.9 | 3,840,226 | 3.5% | Unknown |
| 12.9 | 1,594,995 | 1.5% | Unknown |
| 13.8 | 1,594,995 | 4.1 % | Unknown |
| 17.3 | 4,434,046 | 11.1 % | 2,5-dimethylpyrazine |

As indicated, the volatile compounds identified as being associated with *S. choleraesuis* included alcohols and heterocyclic compounds.

### EXAMPLE 6

The results obtained in Examples 1-5 were analyzed to identify one or more volatile compounds associated with *P. aeruginosa* (gram negative), *E. coli* (gram negative), *S. aureus* (gram positive), *C. albicans* (yeast), and *S. choleraesuis* (gram negative). *P. aeruginosa, E. coli,* and *S. aureus* produced 1-undecene. However, the remaining volatile profiles for these bacteria were significantly different. *P. Aeruginosa* generated a series of tiglic acid esters, sulfur compounds, and esters of small branched acids. *S. Aureus* had a simpler volatile profile, with only two compounds at significantly greater concentration than the nutrient blank. The most prominent peak was 2-acetylthiazole. In addition, *E. coli* volatiles contained only three compounds that were in significantly greater concentration than the nutrient blank. Indole was the prominent peak. The volatile profile for *C. albicans* was significantly different from the volatiles found from *P. aeruginosa, S. aureus,* and *E. coli*. The presence of many impurity peaks in both the transport control and the media control made it difficult to determine what compounds were actually predominately attributable to the *S. choleraesuis* off-gases. However, some compounds found predominately in the *S. choleraesuis* culture included isopropanol, 2,5-dimethyl pyrazine, ethanol, and possibly 3-methyl-1-butanol and cyclohexane. Based on the above, the following volatile compounds were identified:

| **Microorganism** | **Volatile Compound** |
|---|---|
| *E. coli* | Indole |
| *S. aureus* | 2-Acetyl thiazole |
| *P. aurignosa* | Methyl 2-methyl-2-butenoate |
| *C. albicans* | Iso-amyl alcohol |
| *S. choleraesuis* | 2,5-dimethylpyrazine |

The next step was to identify color changing dyes that would be sensitive to the microbial volatiles as a means of generating a visual indicator for the presence of the microorganism. Table 8 lists dyes sensitive to the specific volatile compounds that also yield a color change when exposed to very low concentrations of the targeted compound.

**Table 8: Color Changing Dyes Identified for Volatile Compounds**

| **Microorganism** | **Volatile Compound** | **Color Changing Dye** |
|---|---|---|
| *E. coli* | Indole | Dimethylaminocinnamaldehyde |
| *S. aureus* | 2-Acetyl thiazole | 2,4-dinitrophenylhydrazine |
| *P. aurignosa* | Methyl 2-methyl-2-butenoate | Potassium permanganate |
| *C. albicans* | Iso-amyl alcohol | Ammonium dichromate |
| *S. choleraesuis* | 2,5-dimethylpyrazine | Potassium permanganate |

The selected dyes rapidly change color in the solution phase when exposed to the volatile compounds. Table 9 lists the color changes observed when the dye was reacted with the model compounds.

**Table 9: Visual Color Change Triggered by Volatile Compounds**

| **Microorganism** | **Volatile Compound** | **Initial Color** | **Final Color** |
|---|---|---|---|
| *E. coli* | Indole | Light pink | Deep purple |
| *S. aureus* | 2-Acetyl thiazole | Light yellow | Orange |
| *C. albicans* | Iso-amyl alcohol | Orange | Green |
| *S. choleraesuis* | 2,5-dimethylpyrazine | Light pink | Deep purple |
| *P. aurignosa* | Methyl 2-methyl-2-butenoate | Purple | Brown |

As indicated, the dyes served as effective indicators for the identified volatile compounds.

### EXAMPLE 7

The ability of the dyes selected in Example 6 to react with the respective volatile compound when present in the gas phase was demonstrated. Specifically, solutions of each dye (40 milligrams of dye dissolved in 10 milliliters of acetone or water) were coated onto small strips of a substrate via aliquots dispensed via Pasture dropper. Each substrate was pre-treated with Snowtex^{™} OXS silica nanoparticles and allowed to air dry. The silica nanoparticles increased the surface area of the strips, thereby increasing the exposure of the dye to the volatile compounds. Table 10 reflects the particular solvent and substrate employed.

**Table 10: Solvent and Substrate Used**

| **Dye** | **Solvent** | **Strip Substrate** |
|---|---|---|
| Potassium permanganate | Water | Polypropylene (PP) nonwoven |
| Dimethylamino cinnamaldehyde | Acetone | Cotton |
| Ammonium dichromate | Water | PP nonwoven |
| Dinitrophenylhydrazine | Ethanol | Cotton or PP nonwoven |

Testing was conducted by exposing the dye-coated strips to the headspace for the unique volatile compounds for each microorganism. Specifically, the dye-coated strips were suspended in the vial so as not to touch the liquid at the bottom of the vial. In each case, a color change was observed within 1 minute of exposure to the volatile compounds. A "real world test" was also carried out using a suspension of five *E. coli* bacteria. The viable bacteria suspension (100 microliters) was placed into a scintillation vial and a strip of dye-coated cotton fabric was suspended from the top of the vial. A color change was observed within 1 minute after screwing on the lid. No color change occurred when the same experiment was repeated using the growth media alone. Thus, the indicator dye was clearly able to identify the presence of *E. Coli*.

While the invention has been described in detail with respect to the specific embodiments thereof, it will be appreciated that those skilled in the art, upon attaining an understanding of the foregoing, may readily conceive of alterations to, variations of, and equivalents to these embodiments.

## Claims

1. A method for detecting the presence of a microorganism, the method comprising:
extracting a headspace gas produced by a culture of the microorganism;
analyzing the extracted headspace gas and identifying a volatile compound associated with the microorganism culture;
selecting an indicator that is capable of undergoing a detectable color change in the presence of the identified volatile compound;
applying the indicator to a surface of the substrate to form an indicator strip; and
detecting the microorganism by observing a color change on the indicator strip.

2. The method of claim 1, further comprising applying an additional indicator to the surface of the substrate, the additional indicator being capable of undergoing a detectable color change in the presence of an additional volatile compound, the additional volatile compound being associated with a culture of an additional microorganism.

3. The method of claim 1, wherein the headspace gas is extracted using solid phase microextraction.

4. The method of claim 1 or 3, further comprising contacting the extracted headspace gas with a chromatographic column of a gas chromatograph to separate one or more components of the headspace gas.

5. The method of claim 1, 3, or 4, further comprising subjecting the separated components of the headspace gas to mass spectroscopy to produce a mass spectrum.

6. The method of any of the foregoing claims, wherein the microorganism is a bacteria.

7. The method of claim 6, wherein the microorganism is *P. aeruginosa, E. coli, S. aureus, C. albicans*, or *S. choleraesuis*.

8. The method of claim 6, wherein the microorganism is *P. aeruginosa* and the identified volatile compound is methyl 2-methyl-2-butenoate.

9. The method of claim 6, wherein the microorganism is *E. coli* and the identified volatile compound is indole.

10. The method of claim 6, wherein the microorganism is *S. aureus* and the identified volatile compound is 2-acetyl thiazole.

11. The method of claim 6, wherein the microorganism is *C. albicans* and the identified volatile compound is iso-amyl alcohol.

12. The method of claim 6, wherein the microorganism is *S. choleraesuis* and the identified volatile compound is 2,5-dimethylpyrazine.

13. The method of any of the foregoing claims, wherein the indicator is potassium permanganate, dimethylaminocinnamaldehyde, 2,4-dinitrophenylhydrazine, or ammonium dichromate.

14. A substrate for detecting the presence of multiple microorganisms, the substrate containing at least first and second indicator zones, wherein a first indicator is contained within the first indicator zone in an amount effective to cause a detectable color change upon contact with a first volatile compound produced by a first microorganism, and wherein a second indicator is contained within the second indicator zone in an amount effective to cause a detectable color change upon contact with a second volatile compound produced by a second microorganism, whereby the first and second indicators are selected from the group consisting of potassium permanganate, dimethylaminocinnamaldehyde, 2,4-dinitrophenylhydrazine, and ammonium dichromate.

15. The substrate of claim 14, wherein the first and second volatile compounds are selected from the group consisting of methyl 2-methyl-2-butenoate, indole, 2-acetyl thiazole, iso-amyl alcohol, and 2,5-dimethylpyrazine.

16. The substrate of claim 14 or 15, wherein each indicator is present in an amount from 0.001 wt.% to about 10 wt.% of the substrate.

17. The substrate of any of claims 14 to 16, wherein the substrate further comprises high-surface area particles.

## Patentansprüche

1. Ein Verfahren zum Nachweis des Vorhandenseins eines Mikroorganismus, wobei das Verfahren umfasst:
Entnehmen eines Headspace-Gases, welches durch eine Kultur des Mikroorganismus erzeugt wurde;
Analysieren des entnommenen Headspace-Gases und Identifizieren einer flüchtigen Verbindung, welche der Mikroorganismuskultur zugeordnet ist;
Auswählen eines Indikators, der in der Lage ist, einen nachweisbaren Farbwechsel in Anwesenheit der identifizierten flüchtigen Verbindung auszuführen;
Anwenden des Indikators auf eine Oberfläche des Substrats, um einen Indikatorstreifen zu bilden; und
Nachweisen des Mikroorganismus durch Beobachten eines Farbwechsels auf dem Indikatorstreifen.

2. Das Verfahren gemäß Anspruch 1, welches des Weiteren das Anwenden eines zusätzlichen Indikators auf die Oberfläche des Substrats umfasst, wobei der zusätzliche Indikator in der Lage ist, einen nachweisbaren Farbwechsel in Anwesenheit einer zusätzlichen flüchtigen Verbindung auszuführen, wobei die zusätzliche flüchtige Verbindung einer Kultur eines zusätzlichen Mikroorganismus zugeordnet ist.

3. Das Verfahren gemäß Anspruch 1, wobei das Headspace-Gas unter Verwendung von Festphasenmikroextraktion entnommen wird.

4. Das Verfahren gemäß Anspruch 1 oder 3, welches des Weiteren das Kontaktieren des entnommenen Headspace-Gases mit einer Chromatographiesäule eines Gaschromatographen umfasst, um eine oder mehrere Komponenten des Headspace-Gases zu trennen.

5. Das Verfahren gemäß Anspruch 1, 3 oder 4, welches des Weiteren umfasst, die getrennten Komponenten des Headspace-Gases einer Massenspektrometrie zu unterziehen, um ein Massenspektrum zu erzeugen.

6. Das Verfahren gemäß einem der vorherigen Ansprüche, wobei der Mikroorganismus ein Bakterium ist.

7. Das Verfahren gemäß Anspruch 6, wobei der Mikroorganismus *P. aeruginosa, E. coli, S. aureus, C. albicans* oder *S. choleraesuis* ist.

8. Das Verfahren gemäß Anspruch 6, wobei der Mikroorganismus *P. aeruginosa* ist und die identifizierte flüchtige Verbindung Methyl 2-Methyl-2-Butenoat ist.

9. Das Verfahren gemäß Anspruch 6, wobei der Mikroorganismus *E. coli* ist und die identifizierte flüchtige Verbindung Indol ist.

10. Das Verfahren gemäß Anspruch 6, wobei der Mikroorganismus *S. aureus* ist und die identifizierte flüchtige Verbindung 2-Acetyl-thiazol ist.

11. Das Verfahren gemäß Anspruch 6, wobei der Mikroorganismus *C. albicans* ist und die identifizierte flüchtige Verbindung Isoamylalkohol ist.

12. Das Verfahren gemäß Anspruch 6, wobei der Mikroorganismus *S. choleraesuis* identifizierte flüchtige Verbindung 2,5-Dimethylpyrazin ist.

13. Das Verfahren gemäß einem der vorherigen Ansprüche, wobei der Indikator Kaliumpermanganat, Dimethylaminocinnamaldehyd, 2,4-Dinitrophenylhydrazin oder Ammoniumdichromat ist.

14. Ein Substrat zum Nachweis des Vorhandenseins von mehreren Mikroorganismen, wobei das Substrat mindestens erste und zweite Indikatorbereiche enthält, wobei ein erster Indikator innerhalb des ersten Indikatorbereichs enthalten ist in einer Menge, welche ausreicht, einen nachweisbaren Farbwechsel bei Kontakt mit einer ersten flüchtigen Verbindung zu verursachen, welche von einem ersten Mikroorganismus erzeugt wird, und wobei ein zweiter Indikator innerhalb des zweiten Indikatorbereichs enthalten ist in einer Menge, welche ausreicht, einen nachweisbaren Farbwechsel bei Kontakt mit einer zweiten flüchtigen Verbindung zu verursachen, welche von einem zweiten Mikroorganismus erzeugt wird, wobei die ersten und zweiten Indikatoren ausgewählt sind aus der Gruppe bestehend aus Kaliumpermanganat, Dimethylaminocinnamaldehyd, 2,4-Dinitrophenylhydrazin und Ammoniumdichromat.

15. Das Substrat gemäß Anspruch 14, wobei die ersten und zweiten flüchtigen Verbindungen ausgewählt sind aus der Gruppe bestehend aus Methyl 2-Methyl-2-Butenoat, Indol, 2-Acetyl-thiazol, Isoamylalkohol und 2,5-Dimethylpyrazin.

16. Das Substrat gemäß Anspruch 14 oder 15, wobei jeder Indikator in einer Menge von 0,001 Gew.-% bis ungefähr 10 Gew.-% des Substrats vorhanden ist.

17. Das Substrat gemäß einem der Ansprüche 14 bis 16, wobei das Substrat des Weiteren Teilchen mit einem großen Oberflächenbereich umfasst.

## Revendications

1. Procédé de détection de la présence d'un microorganisme, le procédé comprenant :
l'extraction d'un gaz de tête produit par une culture du microorganisme ;
l'analyse du gaz de tête extrait et l'identification d'un composé volatil associé à la culture du microorganisme ;
la sélection d'un indicateur capable de subir un changement de couleur détectable en présence du composé volatil identifié ;
l'application de l'indicateur à la surface d'un substrat pour former une bande indicatrice ; et
la détection du microorganisme en observant un changement de couleur sur la bande indicatrice.

2. Procédé selon la revendication 1, comprenant, en outre, l'application d'un indicateur supplémentaire à la surface du substrat, l'indicateur supplémentaire étant capable de subir un changement de couleur détectable en présence d'un composé volatil supplémentaire, le composé volatil supplémentaire étant associé à une culture d'un microorganisme supplémentaire.

3. Procédé selon la revendication 1, dans lequel le gaz de tête est extrait en utilisant une microextraction en phase solide.

4. Procédé selon la revendication 1 ou 3, comprenant, en outre, la mise en contact du gaz de tête extrait avec une colonne chromatographique d'un chromatographe opérant en phase gazeuse pour séparer un ou plusieurs composants du gaz de tête.

5. Procédé selon la revendication 1, 3 ou 4, comprenant, en outre, le fait de soumettre les composants séparés du gaz de tête à une spectroscopie de masse pour produire un spectre de masse.

6. Procédé selon l'une quelconque des revendications précédentes, dans lequel le microorganisme est une bactérie.

7. Procédé selon la revendication 6, dans lequel le microorganisme est *P. aeruginosa, E. coli, S. aureus, C. albicans* ou *S. choleraesuis*.

8. Procédé selon la revendication 6, dans lequel le microorganisme est *P. aeruginosa* et le composé volatil identifié est le 2-méthyl-2-buténoate de méthyle.

9. Procédé selon la revendication 6, dans lequel le microorganisme est *E. coli* et le composé volatil identifié est l'indole.

10. Procédé selon la revendication 6, dans lequel le microorganisme est *S. aureus* et le composé volatil identifié est le 2-acétyl thiazole.

11. Procédé selon la revendication 6, dans lequel le microorganisme est *C. albicans* et le composé volatil identifié est l'alcool isoamylique.

12. Procédé selon la revendication 6, dans lequel le microorganisme est *S. choleraesuis* et le composé volatil identifié est la 2,5-diméthylpyrazine.

13. Procédé selon l'une quelconque des revendications précédentes, dans lequel l'indicateur est le permanganate de potassium, le diméthylaminocinnamaldéhyde, la 2,4-dinitrophénylhydrazine ou le dichromate d'ammonium.

14. Substrat pour la détection de la présence de microorganismes multiples, le substrat contenant au moins une première et une seconde zones indicatrices, un premier indicateur étant contenu au sein de la première zone indicatrice en une quantité efficace à provoquer un changement de couleur détectable lors d'un contact avec un premier composé volatil produit par un premier microorganisme, et un second indicateur étant contenu dans une seconde zone indicatrice en une quantité efficace à provoquer un changement de couleur détectable lors d'un contact avec un second composé volatil produit par un second microorganisme, les premier et second indicateurs étant sélectionnés dans le groupe consistant en le permanganate de potassium, le diméthylaminocinnamaldéhyde, la 2,4-dinitrophénylhydrazine et le dichromate d'ammonium.

15. Substrat selon la revendication 14, dans lequel les premier et second composés volatils sont sélectionnés dans le groupe consistant en le 2-méthyl-2-buténoate de méthyle, l'indole, le 2-acétyl thiazole, l'alcool isoamylique et la 2,5-diméthylpyrazine.

16. Substrat selon la revendication 14 ou 15, dans lequel chaque indicateur est présent en une quantité comprise entre 0,001 % en poids et environ 10 % en poids par rapport au substrat.

17. Substrat selon l'une quelconque des revendications 14 à 16, ledit substrat comprenant, en outre, des particules à grande surface massique.
